# EUROPEAN PATENT APPLICATION

(11) **EP 1 785 152 A1**
(43) Date of publication of application: **16.05.2007**
(21) Application number: 06291750.5
(22) Date of filing: 10.11.2006
(51) Int. Cl.: A61L 9/015, A61L 9/22, F24F 3/16

(54) **Sterilizer and method for controlling the same**

(30) Priority: 11.11.2005 KR 20050107909
(71) Applicant: LG Electronics Inc., Yongdungpo-gu Seoul (KR)
(72) Inventor: Choi, In Ho, Goonpo-si Kyunggi-do (KR); Lee, Ju Youn, Dongjak-ku Seoul (KR); Kim, Ho Jung, Boopyung-ku Incheon-si (KR)
(74) Representative: Verdure, Stéphane

(57) **Abstract**

Provided is a sterilizer and a method for controlling the sterilizer. A sterilizer includes a sensor unit measuring a growing condition of microorganism, a sterilizing unit generating a microbicide for sterilizing the microorganisms, and a control unit controlling an operational condition of the sterilizing unit according to a growing condition of microorganisms detected by the sensor unit.

## Description

### BACKGROUND OF THE INVENTION

### Field of the Invention

The present invention relates to a sterilizer, and more particularly, to a sterilizer and a method for controlling the same, which can generate a proper amount of ozone according to the growing condition of microorganisms, thereby minimizing the power consumption and preventing the sterilizer from being harmful to human.

### Description of the Related Art

Generally, a variety of methods such as a high pressure/temperature treating method, a chemical treating method using ethylene oxide, an ultraviolet treating method, and a sterilizing method using plasma discharge has been used to sterilize microorganisms such as mold and viruses.

The high pressure/temperature treating method uses a high pressure/temperature steam. This method may corrode metal and may not be applied to plastic.

The chemical treating method using the ethylene oxide is excellent in a sterilizing treatment at a low temperature. However, the ethylene oxide is an inflammable and toxic material. Therefore, it must be very carefully treated. In addition, the toxic material may remain on the treated object.

The ultraviolet treating method uses an ultraviolet lamp. A merit of this method is that it is convenient to use. However, a portion where the lamp does not directly contact is not sterilized.

Therefore, the sterilizing method using the plasma discharge has been widely used. This method uses a high electron energy generated by a dielectric barrier discharge (DBD).

That is, this method generates OH radical, negative ions, and ozone using the high electron energy to sterilize bacteria or mold and break the chemical connection chain of the volatile organic compositions.

However, due to the high oxidation of the OH radical, negative ions and ozone generated by the plasma discharge, when these components are exposed to an enclosed environment, thereby may be harmful to human. Many countries including Korea propose a guideline of the ozone. In Korea, it is regulated that it cannot above 50ppb (parts per billion) for 24 hours.

Meanwhile, in the convention sterilizer, a contact electric power must be continuously applied to generate the ozone. In this case, the ozone is satirized in the enclosed interior space and thus harmful to human.

In addition, as the electric power is continuously applied, the power consumption increases and the sterilizer may be excessively operated, thereby increasing the maintenance costs of the sterilizer.

### SUMMARY OF THE INVENTION

Accordingly, the present invention is directed to a sterilizer and a method for controlling the same that substantially obviates one or more problems due to limitations and disadvantages of the related art.

An object of the present invention is to provide a sterilizer and a method for controlling the same, which can variably control the control module, i.e., an amount of ozone and an airflow rate according to a growing condition of microorganisms.

Another object of the present invention is to provide a sterilizer and a method for controlling the same, which can variably control the operation of the sterilizer according to the according to a growing condition of microorganisms, thereby preventing the sterilizer from excessively operating and from being harmful to human by the ozone saturation.

Still another object of the present invention is to provide a sterilizer and a method for controlling the same, which can minimize the power consumption by properly adjusting the number of on/off times and on/off duration of the electric power, thereby reducing the maintenance costs of the sterilizer.

Additional advantages, objects, and features of the invention will be set forth in part in the description which follows and in part will become apparent to those having ordinary skill in the art upon examination of the following or may be learned from practice of the invention. The objectives and other advantages of the invention may be realized and attained by the structure particularly pointed out in the written description and claims hereof as well as the appended drawings.

To achieve these objects and other advantages and in accordance with the purpose of the invention, as embodied and broadly described herein, there is provided a sterilizer including: a sensor unit measuring a growing condition of microorganism; a sterilizing unit generating a microbicide for sterilizing the microorganisms; and a control unit controlling an operational condition of the sterilizing unit according to a growing condition of microorganisms detected by the sensor unit.

In another aspect of the present invention, there is provided a sterilizer including: a sensor unit measuring a growing condition of microorganism; a sterilizing unit generating ozone for sterilizing the microorganisms; and a control unit determining an operational condition according to a growing condition of microorganisms detected by the sensor unit and controlling the sterilizing unit according to the determined condition; and a blower unit for distributing the ozone generated into an interior space.

In still another aspect of the present invention, there is provided a method of controlling a sterilizer, including: determining an operational condition of a sterilizing unit according to a growing condition of microorganisms detected; and operating the sterilizing unit to generate ozone according to the determined operational condition.

As proposed above, an amount of ozone and an airflow rate can vary according to a growing condition of microorganisms.

In addition, since the operational condition of the sterilizing unit is variably set according to the growing condition of the microorganisms, the saturation of the ozone in the interior space can be prevented, thereby preventing the ozone from being harmful to human.

Furthermore, the operation of the sterilizer can be variably controlled according to the according to a growing condition of microorganisms, thereby preventing the sterilizer from excessively operating and from being harmful to human by the ozone saturation.

Still another object of the present invention is to provide a sterilizer and a method for controlling the same, which can minimize the power consumption by properly adjusting the number of on/off times and on/off duration of the electric power, thereby reducing the maintenance costs of the sterilizer.

It is to be understood that both the foregoing general description and the following detailed description of the present invention are exemplary and explanatory and are intended to provide further explanation of the invention as claimed.

### BRIEF DESCRIPTION OF THE DRAWINGS

The accompanying drawings, which are included to provide a further understanding of the invention and are incorporated in and constitute a part of this application, illustrate embodiment(s) of the invention and together with the description serve to explain the principle of the invention. In the drawings:

Fig. 1 is a view illustrating a growing condition of microorganisms;

Fig. 2 is a block diagram illustrating a sterilizer according to an embodiment of the present invention;

Fig. 3 is a view illustrating an operational condition of a sterilizer according to a temperature and a humidity;

Fig. 4 is a view illustrating an mount of ozone introduced into an interior space by a sterilizing unit according to an embodiment of the present invention;

Figs. 5 through 7 are views illustrating an on/off duration of power applied to a sterilizer; and

Fig. 8 is a view illustrating a method for controlling a sterilizer according to an embodiment of the present invention.

### DETAILED DESCRIPTION OF THE INVENTION

Reference will now be made in detail to the preferred embodiments of the present invention, examples of which are illustrated in the accompanying drawings. The invention may, however, be embodied in many different forms and should not be construed as being limited to the embodiments set forth herein; rather, these embodiments are provided so that this disclosure will be thorough and complete, and will fully convey the concept of the invention to those skilled in the art.

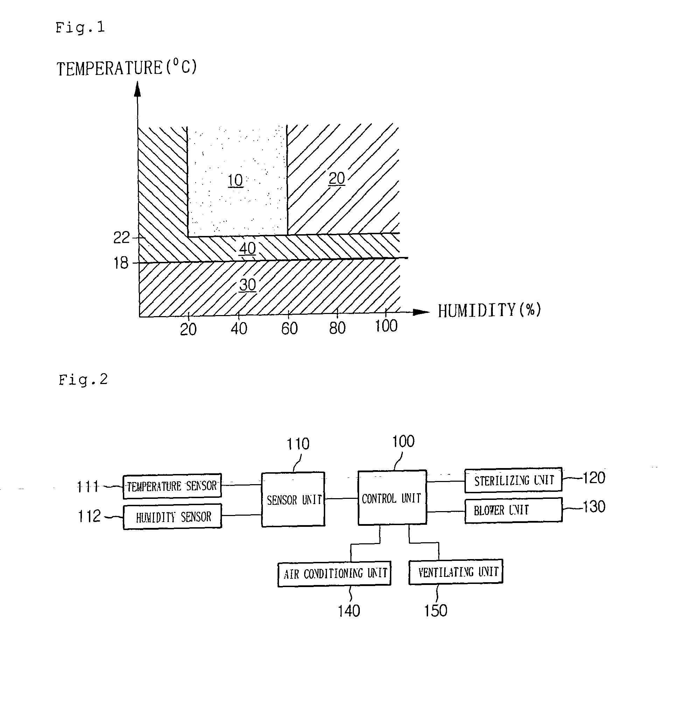

Fig. 1 is a view illustrating a growing condition of microorganisms.

In the growing condition of FIG. 1, an example where mold and virus are used as viruses and the growing condition is divided into four regions is illustrated. However, the present invention is not limited to this example.

That is, a generation amount of the mold and virus varies depending on the temperature and humidity and quickly increases in 60%RH state. During the growth, virus spores are dispersed into the air to cause allergy, atopy, asthma, headache, and the like.

Therefore, the growing conditions can be divided using the temperature and humidity. The following will describe the growing condition of the mold and virus using the exemplary temperature and humidity.

The region where the temperature is equal to or less than 18°C is a virus sustain region 30, in which the activity and generation amount of the virus is not significantly increased but the virus can grow.

In addition, the region where the temperature is equal to or less than 22°C and the humidity is 20~60% is a mold sustain region 10 in which the activity and generation amount of the mold is not significantly increased but the mold grow.

The region where the temperature is equal to or less than 22°C and the humidity is equal to or greater than 60% is a mold increase region where the activity and generation amount of the mold is significantly increased and thus the interior air quality may be steeply deteriorated.

The region where the temperature is equal to or greater than 22°C and the humidity is equal to or less than 20% is a virus increase region where the activity and generation amount of the virus is increased and thus the interior air quality may be deteriorated.

Fig. 2 is a block diagram of a sterilizer according to an embodiment of the present invention.

Referring to Fig. 2, a sterilizer of this embodiment includes a sensor unit 110 for detecting a growing condition of microorganisms of the interior space, a control unit 100 for allowing a proper amount of ozone to be generated according to a temperature/humidity detected by the sensor unit 11, a sterilizing unit 120 for generating the ozone according to the control of the control unit 100, a blower unit 130 for dispersing the ozone into the interior space according to the control of the control unit 100. The blower unit varies the airflow rate according to the control of the control unit 100.

In addition, the sterilizer may further include an air conditioning unit 140 for heating and cooling the interior space and a ventilating unit 150 for ventilating the interior space.

The sensor unit 110 includes a temperature sensor 111 for detecting an interior temperature and a humidity sensor 112 for detecting an interior humidity. The temperature and humidity sensors 111 and 112 may be placed at proper locations where the mold and virus can be grown.

In addition, the control unit 100 reads the temperature and/or humidity detected by the sensor unit 110 and controls an amount of the ozone supplied to the interior space and an airflow rate.

The control unit 100 controls a ratio between a power-On duration and a power-Off duration to properly set the amount of the ozone.

The control unit 100 controls a dispersing speed of the ozone into the interior space by controlling the airflow rate generated by the blower unit 130.

That is, the control unit 100 can read the temperature and/or humidity of the interior space, which is detected by the sensor unit 110, while the ozone is supplied into the interior space by the sterilizing unit 120.

In this case, when the microorganism growing condition is different from a condition of the initial driving of the sterilizer 120, the control unit 100 can reset the driving condition of the sterilizer 120 and the blower unit 130. Accordingly, even when the microorganism growing condition of the interior space varies, the control unit 100 can control a generation amount of the ozone in response to the varied condition, thereby preventing the excessive ozone from being harmful to human.

Here, the sterilizer unit 120 may be a device that can generate the ozone using the plasma discharge. Particularly, the sterilizer unit 120 may be a device that can generate the OH radical, negative ions, ozone, and the like using the DBD.

The blower unit 130 functions to generate a wind pressure using a fan so that the ozone generated by the sterilizer 120 is supplied to the interior space. As the rpm of the fan varies, the wind pressure varies.

The air conditioning unit 140 functions to heat and cool the interior space, including a compressor, a heat exchanger, and an expander. The heating and cooling of the interior space by the air conditioning unit 140 may be controlled by the control unit 100.

The ventilating unit 150 functions to supply external fresh air to the interior space while exhausting polluted air to an external side. The ventilating unit 150 is also controlled by the control unit 100.

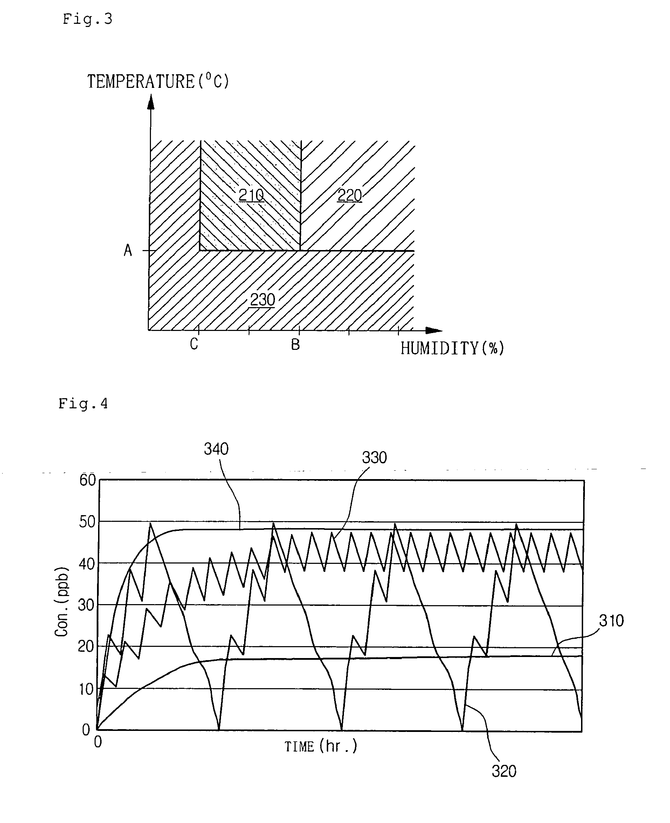

Fig. 3 is a view illustrating an operational condition of the sterilizer.

Referring to Fig. 3, the sterilizer of the present invention is variably controlled according to conditions of regions divided according to the temperature and humidity of the interior space. That is, according to the growing condition of the microorganisms, a generation amount of the ozone by the sterilizing unit 120 varies.

That is, when the temperature measured by the sensor unit 110 is equal to or greater than A°C and the humidity is C-B%, the interior space can be defined as a first region 210 where the growth of the mold can be stopped. In the first region 210, the activity and generation of the mold is not significantly increased but the mold can be still grown.

Therefore, when it is determined that the interior space is defined as the first region 210, the control unit 100 significantly reduces the voltage applied to the sterilizing unit 120 to reduce the power consumption and prevent the ozone from being excessively supplied into the interior space.

In addition, when the temperature detected by the sensor unit 110 is equal to or greater than A°C and the humidity detected by the sensor unit 110 is B%, the interior space can be defined as a second region 220. In the second region 220, the activity or generation amount of the mold can be steeply increased and thus the air quality of the interior space is steeply deteriorated.

Therefore, when it is determined that the interior space is defined as the second region 220, the control unit 100 controls the sterilizing unit 120 and the blower unit 130 such that a large amount of ozone can be supplied into the interior space in a short time.

Meanwhile, the interior space may be further defined as a third region where the temperature detected by the sensor unit 110 is equal to or greater than A°C and the humidity is less than C% and a fourth region where the temperature detected by the sensor unit 110 is less than A°C.

In the third region 230, the activity and generation amount of the virus may be increased and thus the air quality may be deteriorated.

Therefore, when it is determined that the interior space is defined as the third region 230, the control unit 100 controls the sterilizing unit 120 such that a proper amount of the ozone can be supplied by adjusting a ratio between a power-on duration and a power-off duration of the power applied to the sterilizing unit 120.

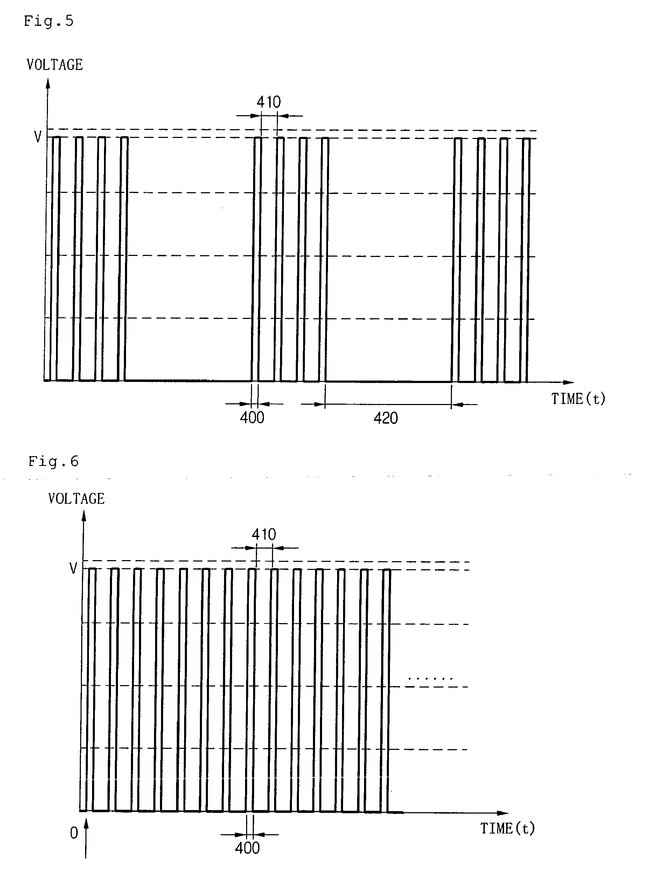

Fig. 4 is a view illustrating an amount of ozone introduced into an interior space by a sterilizing unit according to an embodiment of the present invention and Figs. 5 through 7 are views illustrating an on/off duration of power applied to a sterilizer.

Referring to Figs. 4 through 7, the control of the sterilizing unit 120 of the present embodiment of the present invention is realized by adjusting a ratio between the power-on duration and the power-off duration of the electric power applied to the sterilizing unit 120. Particularly, the power applied to the sterilizing unit 120 is intermittently turned on.

In one embodiment, when the temperature and humidity that are detected by the senor 110 correspond to the first region 210, the sterilizing unit 120 is operated with a first mode 310 so that an amount of the ozone in the interior space can be maintained at 18ppb as time goes.

When the temperature and humidity that are detected by the senor 110 correspond to the third region 230, the sterilizing unit 120 is operated with a third mode 330 so that an amount of the ozone in the interior space can be maintained at 38048ppb as time goes.

That is, when the sterilizing unit 120 operates with the first mode 310 or the third mode 330, the power-on duration is intermittently realized as shown in Fig. 6.

When the sterilizing unit 120 operates with the first mode 310, an amount of the ozone supplied to the interior space is less than that of the third mode 330, and thus the power applied to the sterilizing unit 120 in the third mode 33 is set to be less than that of the first mode.

Meanwhile, when the sterilizing unit 120 operates with the first mode 310 or the third mode 330, the ratio between the power on-time 410 and the power-off time 400 is set as follows.

When the sterilizing unit 120 operates with the first mode 310, the ratio of the power-on time 410 to the power-off time 400 is set at 0.2±0.05. This is for maintaining the amount of the ozone at 18ppb as shown in Fig. 4.

When the sterilizing unit 120 operates with the third mode 330, the ratio of the power-on time 410 to the power-off time 400 is set at 0.1±0.05. This is for maintaining the amount of the ozone at 48ppb as shown in Fig. 4.

When the temperature and humidity that are detected by the senor 110 correspond to the second region 220, the sterilizing unit 120 is operated with the second d mode 320 so that a maximum generation amount of the ozone supplied to the interior space can be 50ppb with a predetermined cycle.

That is, when the sterilizing unit 120 operates with the second mode 310 or the third mode 330, the power-on and power-off durations 410 and 420 is intermittently realized as shown in Fig. 5.

Then, after the power-on duration 41 and power-off are performed by a predetermined times, a break duration 420 where the power-off duration 400 is continuously maintained is provided, thereby completing one cycle.

When the sterilizing unit 120 operates with the second mode 320, the ratio of the power-on time 410 to the power-off time 400 is set at 0.3±0.05. This is for that the increase and decrease of the generation of the ozone supplied to the interior space can be repeated with a predetermined cycle and thus the maximum amount of the ozone can be about 50ppb as shown in Fig. 4.

In addition, when the sterilizing unit 120 operates with the second mode 320, the blower unit 130 is set to increase the wind pressure. This is for supplying a large amount of the ozone to the interior space in the second mode 320 in short time. This can be realized by increasing the rpm of the fan provided in the blower unit 130.

The first through third modes 310, 320, 330 are divided according to the activity and generation amount of the microorganism. That is, as the activity and generation amount of the microorganisms increase, the power-on duration 410 of the power applied to the sterilizing unit 120 is set to be relatively high.

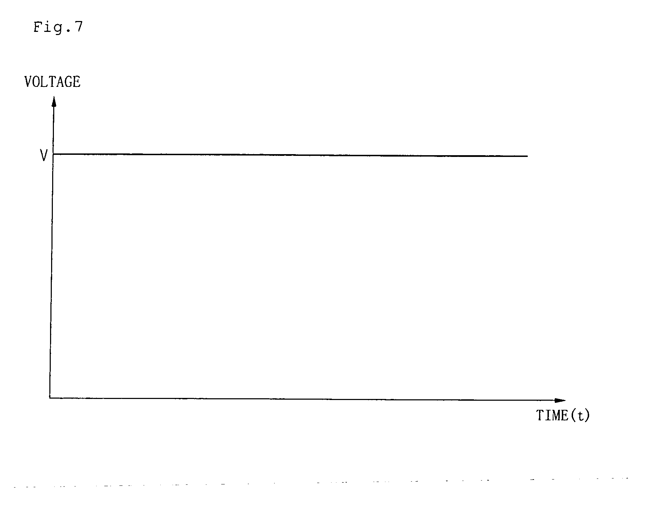

In addition to the intermittent setting of the on/off durations 400 and 41 of the power applied to the sterilizing unit 120, as shown in Fig. 7, the ozone may be generated by continuously applying a direct current power.

That is, when the When the sterilizing unit 120 operates with the fourth mode 340, this mode may be a manual mode where the amount of the ozone is controlled by a user. In this case, the amount of the ozone supplied to the interior space is continuously increased.

In order to prevent the ozone from be excessively supplied in the fourth mode 340, the ventilating unit 150 ventilates the interior space.

To achieve this, the control unit 100 operates the ventilating unit 150 after the sterilizing unit 120 operates for a predetermined time, thereby reducing the amount of the ozone in the interior space. In addition, the control unit 10 can detect the amount of the ozone in the interior space using the sensor. When the amount of the ozone reaches a preset level, the control unit 100 operates the ventilating unit 150 to reduce the amount of the ozone in the interior space.

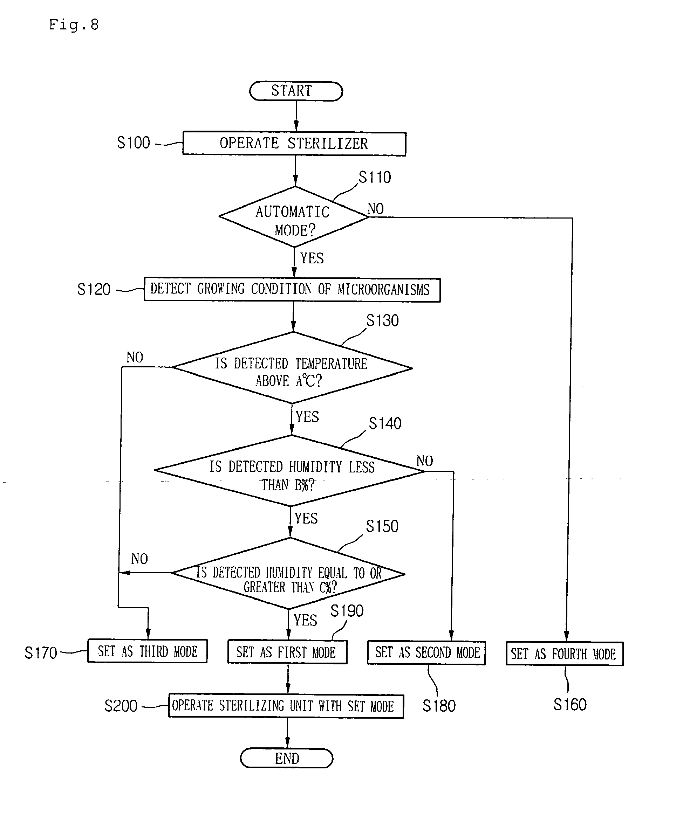

Fig. 8 is a view illustrating a method for controlling a sterilizer according to an embodiment of the present invention.

Referring to Fig. 8, when the sterilizing unit operates (S100), it is determined if the operation of the sterilizing unit is set as an automatic mode (S110). The automatic mode is for intermittently applying the power to the sterilizing unit, thereby reducing the power consumption of the sterilizing unit and preventing the ozone from being excessively supplied into the interior space.

If the operation mode of the sterilizing unit is not set at the automatic mode but the manual mode, the control unit 100 continuously applies the direct current power to the sterilizing unit 120 so that the sterilizing unit 120 operates with the fourth mode 340 (S160).

However, when the operation mode of the sterilizing unit is set at the automatic mode, the temperature and humidity of the interior space is detected by the sensor unit 110 (S120), and the control unit 100 reads the detected temperature and humidity.

Then, the control unit 100 determines if the temperature detected by the sensor unit 110 is above A°C (S130). When the detected temperature is less than A°C, the sterilizing unit 120 is set to operate with the third mode 330 (S170).

When the detected temperature is equal to or greater than A°C, it is determined if the humidity detected by the sensor unit 110 is less than B% (S140). When the detected humidity is less than B%, the control unit 110 sets the sterilizing unit 120 such that the sterilizing unit 120 operates with the second mode 320 (S180).

When the detected humidity is less than B%, it is further determined if the humidity is equal to or greater than C% that is less than B% (S150). When the detected humidity is less than C%, the control unit 100 sets the sterilizing unit 120 such that the sterilizing unit 120 operates with the third mode 330 (S170). In addition, when the measured humidity is equal to or greater than C%, the sterilizing unit 120 is set to operate with the first mode 310 (S190).

As described above, when the sterilizing unit is set to operate with the automatic mode, the operation mode (The first through third modes) of the sterilizing unit 120 is set and the sterilizing unit 120 is automatically operated according to the mode set (S200).

By the above-described method, the sterilizing unit 120 variably operates according to the temperature and humidity of the interior space, thereby reducing the power consumption and maintaining a proper amount of the ozone in the interior space.

In addition, the sterilizing unit cooperates with the air conditioning unit that heats, cools, and ventilates the interior space. In this case, the sterilizing function for killing the microorganism such as mold and viruses as well as the air conditioning function can be realized.

It will be apparent to those skilled in the art that various modifications and variations can be made in the present invention. Thus, it is intended that the present invention covers the modifications and variations of this invention provided they come within the scope of the appended claims and their equivalents.

## Claims

1. A sterilizer for killing microorganisms contained in air, **characterized in that** the sterilizer comprises:
a sensor unit measuring a growing condition of microorganism;
a sterilizing unit generating a microbicide for sterilizing the microorganisms; and
a control unit controlling an operational condition of the sterilizing unit according to a growing condition detected by the sensor unit.

2. The sterilizer according to claim 1, **characterized in that** the growing condition of the microorganisms is determined by a temperature and a humidity.

3. The sterilizer according to claim 1, **characterized in that** a ratio between an on-during and an off-duration of a power applied to the sterilizing unit varies according to the growing condition of the microorganisms detected by the sensor unit.

4. The sterilizer according to claim 1, **characterized in that** the sensor unit comprises a temperature sensor detecting the temperature and a humidity sensor detecting the humidity

5. The sterilizer according to claim 1, **characterized in that** the sterilizer is applied to an air conditioner heating and cooling the air.

6. The sterilizer according to claim 1, **characterized in that** the sterilizer further comprises a blower unit for dispersing the microbicide from the sterilizing unit into the interior space.

7. The sterilizer according to claim 1, **characterized in that** the microbicide includes ozone generated by a plasma discharge.

8. The sterilizer according to claim 1, **characterized in that** the sterilizer further comprises an ozone detecting sensor detecting an amount of ozone distributed in the interior space and a ventilating unit operated according to the amount of the ozone detected by the ozone detecting sensor.

9. The sterilizer according to claim 1, **characterized in that** the sterilizer further comprises an air conditioning unit cooperating with the sterilizing unit and controlled by the control unit.

10. The sterilizer according to claim 1, **characterized in that** the control unit controls the sterilizer such that the sterilizer operates with an automatic or manual mode.

11. The sterilizer according to claim 10, **characterized in that**, in the automatic mode, the on/off duration of the sterilizing is periodically or non-periodically realized by the control unit.

12. The sterilizer according to claim 10, **characterized in that**, in the manual mode, the on-duration of the sterilizing unit is continuously maintained.

13. A method of controlling a sterilizer for killing microorganisms, **characterized in that** the method comprises:
determining an operational condition of an sterilizing unit according to a growing condition of microorganisms detected; and
operating the sterilizing unit to generate ozone according to the determined operational condition.

14. The method according to claim 13, **characterized in that** the growing condition of the microorganisms is determined depending on a temperature and a humidity.

15. The method according to claim 13, **characterized in that** an on/off cycle or on/off duration of the sterilizing unit varies according to the growing condition of the microorganisms.

16. The method according to claim 13, **characterized in that** the growing condition of the microorganisms are continuously detected during the operation of the sterilizing unit to vary the operational condition of the sterilizing unit.

17. The method according to claim 13, further comprising operating a blower unit so that the ozone generated from the sterilizing unit into the interior space.

18. The method according to claim 17, **characterized in that** a generation amount of the ozone and wind pressure of the blower unit vary according to the growing condition of the microorganisms.

19. The method according to claim 13, **characterized in that** a sterilizing process of the sterilizing unit is performed by synchronizing with an air conditioner performing the heating and cooling.
